(19) Europäisches Patentamt
European Patent Office
Office européen des brevets

(11) Publication number: **0 180 675**
**A1**

# (12) EUROPEAN PATENT APPLICATION

(21) Application number: **84307780.1**

(22) Date of filing: **09.11.84**

(51) Int. Cl.4: **C 07 C 51/41**
C 07 C 53/128
//C10G11/02

(43) Date of publication of application:
**14.05.86 Bulletin 86/20**

(84) Designated Contracting States:
**DE GB IT NL**

(71) Applicant: **ATLANTIC RICHFIELD COMPANY**
**515 South Flower Street**
**Los Angeles California 90071(US)**

(72) Inventor: **Miller, Richard F.**
**18702 Flax Bourton Close**
**Humble Texas 77338(US)**

(72) Inventor: **Link, John**
**19506 Shinwood Drive**
**Humble Texas 77338(US)**

(74) Representative: **Cropp, John Anthony David et al,**
**MATHYS & SQUIRE 10 Fleet Street**
**London, EC4Y 1AY(GB)**

(54) **Process for the preparation and use of organic antimony cracking catalysts.**

(57) Antimony tricarboxylates of higher carboxylic acids and suitable for use in the passivation of metal contaminants which interfere with the functioning of zeolite-containing hydrocarbon cracking catalysts in a hydrocarbon cracking process, can be prepared in high yields by (a) reacting antimony oxide with an anhydride of a lower organic acid, (b) reacting the product from (a) with at least one higher carboxylic acid at a temperature sufficient to vaporise volatile material and removing the vaporised volatile material, (c) optionally simultaneously passing a stream of an inert gas through the reaction mixture, and (d) recovering the antimony tricarboxylate of said higher carboxylic acid.

EP 0 180 675 A1

Croydon Printing Company Ltd.

This invention relates to a method of producing antimony tricarboxylates of higher carboxylic acids and to the use thereof as passivating agents in hydrocarbon cracking processes employing zeolite-containing cracking catalysts.

Catalysts used in cracking hydrocarbons can become contaminated and poisoned by accumulation in the catalyst of metals such as nickel, vanadium, iron, copper and cobalt which are present in the hydrocarbon feedstocks. The detrimental effects of those metals can be mitigated and reversed by use of certain organo-antimony compounds as catalyst passivators. Among those organo-antimony compounds are antimony tricarboxylates. The present

invention is an improved method of preparing antimony tricarboxylates in exceedingly high yields.

## Background of The Invention

Certain antimony compounds are known to be used to treat those cracking catalysts conventionally employed in the catalystic cracking of hydrocarbons for the production of gasoline, motor fuel, blending components and light distillates. These conventional cracking catalysts generally contain silica, or silica-alumina. Such materials are frequently associated with zeolitic materials. These zeolitic materials can be naturally occurring, or they can be produced by conventional ion exchange methods such as to provide metallic ions which improve the activity of the catalyst.

While the presence of certain metals can be beneficial, the presence of others in the catalyst is detrimental. It is well known that varying amounts of metals such as nickel, vanadium and iron cause deterioration of the cracking catalyst during the cracking process. In fact, some oils contain these metals in such a high concentration that they cannot be economically cracked into gasoline and other fuels. The metals accumulate on the cracking catalyst and cause increased hydrogen production and coke laydown on the cracking catalyst, thereby adversely affecting the yield of desired products.

It has heretofore been proposed that those deleterious metals can be passivated by treating the contaminated catalyst with compounds containing antimony, tin, indium or bismuth (see U.S. Patent No. 4,257,919).

Antimony compounds are particularly useful as passivating agents and use of a wide variety of both organic and inorganic antimony compounds has been proposed for that purpose (see U.S. Patent Nos. 4,111,845 and 4,153,536). Among the organic antimony compounds proposed are antimony tricarboxylates such as antimony tridodecanoate and antimony trioctadecanoate.

Prior art processes for antimony tricarboxylate preparation involve a direct reaction between antimony oxide and a carboxylic acid anhydride. Nerdel et al (J.Chem.Ber., 90, 598 (1957)) teach that antimony triacetate or antimony tribenzoate can be prepared by reacting antimony oxide with acetic anhydride or benzoic anhydride, respectively.

Ventura et al (U.S. Pat. 3,803,193) disclose reacting antimony tricarboxylates with alcohols to produce antimony trialkoxides. Ventura indicates that the tricarboxylate can be prepared by reacting antimony oxide with an organic acid anhydride directly or in some instances with an acid directly. The acid by-product of the tricarboxylate-alcohol reaction is taught to be neutralized with ammonia and removed as ammonium acetate solid.

The direct production of antimony tricarboxylates by the prior art methods of direct reaction between antimony oxide and the desired acid anhydride is not satisfactory for producing higher tricarboxylates because the higher anhydrides are not readily available and are synthetically prepared only with great difficulty.

Accordingly, it is an object of the present invention to provide a process for preparation of higher tricarboxylates of antimony which uses readily available reactants and affords high yields of the desired tricarboxylates. It is another object of the invention to provide a synthetic method of preparation wherein the antimony tricarboxylate produced is substantially free of deleterious impurities without extensive purification and has a high level of thermal stability. These and other objects, aspects and advantages of the present invention will become apparent to those skilled in the art from the following description of the invention.

## Summary of The Invention

This invention relates to a highly effective and efficient method of preparing higher antimony tricarboxylates in high yields comprising the steps of (a) reacting antimony oxide with an anhydride of a lower organic acid, (b) reacting the product from a) with at least one higher carboxylic acid at an elevated temperature sufficient to vaporize and remove volatile material, while optionally simultaneously passing a stream of an inert gas through the reaction mixture, and (c) recovering the antimony tricarboxylate of said higher carboxylic acid.

The antimony higher carboxylates can be advantageously used as a cracking catalyst component and result in an unexpected efficiency in laydown efficiency in cracking.

## Detailed Description

According to the present invention, higher antimony tricarboxylates are produced from higher carboxylic acids by

a) reacting antimony oxide with an anhydride of a lower organic acid according to the following equation:

$$Sb_2O_3+3R_1-O-R_1 \text{ ------> } 2Sb(OR_1)_3$$

b) reacting the product from a) with at least one higher carboxylic acid at temperatures sufficiently high to vaporize and expel volatile by products of the reaction according to the equation

$$Sb(OR_1)_3+3R_2COOH \text{ -----> } Sb(OOC.R_2)_3 + 3R_1-OH$$

c) while the reaction proceeds, optionally passing a stream of inert gas through the reaction mixture,

and d) recovering the antimony tricarboxylate of said higher carboxylic acid.

The anhydrides of lower organic acids employed in step a) are those anhydrides which are commercially available or readily prepared. Thus $R_1$ in the equation of step a) has up to five carbon atoms. Preferably $R_1$ is alkanoyl of up to five carbon atoms and the lower organic acid anhydrides include acetic anhydride, propionic anhydride, butyric anhydride and pentanoic anhydride. Acetic anhydride is the especially preferred anhydride of a lower organic acid.

The reaction of step a) can be conducted either with a solvent or without a solvent. When conducted with a solvent, the solvent must be inert to both reactants and the reaction product. A suitable solvent is a hydrocarbon having a boiling point of at least 120°C and includes xylene, toluene, cumene, kerosene, and so forth.

Although an elevated temperature is used to increase reaction speed, the temperature of the step a) reaction is not critical. A suitable temperature range is from 100° to 140°C. A temperature of about 120°C results in a reaction time of about 3 to 4 hours between antimony oxide and acetic anhydride.

The reaction product from step a) is reacted with one or more higher carboxylic acids. Higher carboxylic acids are those acids which have at least six carbon atoms. There is no criticality on the upper limit for the number of carbon atoms which is dictated only by practicality. Preferably, $R_2$ in the equation of step b) is alkyl having from five to about 24 carbon atoms. More preferably, $R_2$ is straight chain or branched chain alkyl having seven to eleven carbon atoms.

As illustrated above by the equation of the step (b) reaction, the reaction by-product of step (b) is the lower organic acid which corresponds to the lower acid anhydride employed in step (a). Thus when acetic anhydride is a reactant in step (a), acetic acid would be the by-product in step (b). In conducting step (b), a technical grade neodecanoic acid (an isomer mixture) is an especially suitable higher carboxylic acid. Such

technical grade neodecanoic acid is mixed with the product of step (a) and the temperature of the mixture is increased until the by-product lower organic acid is evolved as a vapor. When acetic anhydride is a reactant in step (a), evolution of acetic acid vapor is observed in step (b) at a temperature of about 150°C. The temperature is continually increased until the reaction is substantially complete and the upper temperature can be about 300°C. When the reaction for step (b) is substantially complete, it is advantageous to reduce the pressure of the reaction in order to produce as complete removal as possible of by-product lower organic acid and the attendant completeness of reaction. Of course, it is possible to conduct the entirity of reaction step (b) under a sub-atmospheric pressure. The degree of pressure reduction is not particularly critical and its choice depends in part on the physical properties (e.g., boiling point) of the particular by-product species being removed. Thus a convenient pressure reduction can be easily determined by those having ordinary skill in the art.

The high temperature used to drive the reaction of step (b) to completeness also serves to eliminate excess and unreacted neodecanoic acid which is also vaporized. At the completion of the reaction of step (b), the product remaining in the reactor is antimony tricarboxylate of mixed neodecanoate isomer ligands essentially free of deleterious impurities which can be used as a hydrocarbon cracking catalyst passivator without

further purification. The recovery of the product therefore involves merely removing said antimony tricarboxylate from the reactor after completion of step (b).

Neodecanoic acid is a ten-carbon acid having a substantial number of theoretical position isomers and said acid is generically represented as follows:

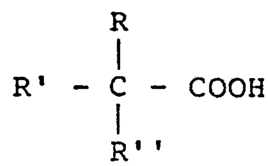

wherein the sum of the carbon atoms of R, R' and R'' is eight alkyl carbon atoms. Although many isomers of neodecanoic are possible, one antimony tricarboxylate preferred for the present invention is produced from a technical grade neodecanoic acid produced by Exxon Chemical Company and which is a highly branched multi-isomer mixture with a typical hydrocarbon odor and a melting point of less than -40°C.

Another preferred higher carboxylic acid reactant for step (b) is a mixture of 2-ethyl hexanoic acid and neodecanoic acid. The molar ratio of 2-ethyl hexanoic acid to neodecanoic acid is about 2:1 so that the resulting antimony tricarboxylate has two ligands of 2-ethylhexanoate and one of neodecanoate.

Of course, other higher carboxylic acids as previously defined as well as mixtures thereof prepared by steps (a) through (c) described above are suitable.

The yield of the desired antimony tricarboxylate of higher carboxylic acids can be dramatically increased by passing an inert gas through the reaction mixture while the product of step a) is reacted with a higher carboxylic acid. One method of passing the inert gas through the reaction mixture is to bubble the gas through the reaction mixture by use of a subsurface sparge. Other means for passing the gas through the mixture will be readily apparent to those skilled in the art and any means for gas-liquid contact is suitable.

The inert gas useful in increasing the yield of antimony tricarboxylates according to this invention is any gas which will not react with a component of the reaction mixture of step b) and which will not leave a possibly deleterious residual substance in the antimony tricarboxylate product. Suitable inert gases include nitrogen, argon, helium, and neon. Nitrogen is preferred.

The flow rate of the inert gas is not critical and its magnitude is limited by practical considerations. For example, the volatile by-product of step b) is generally removed by distillation, e.e., the vapors are condensed and removed. An excessive inert gas flow rate would promote overloading the distillation condenser and thereby impede by-product removal as condensate. Also, an excessive flow rate might cause excessive frothing of the reaction mixture which would not be desired. A suitable inert gas flow rate can easily be determined by those skilled in the art by balancing the gas flow rate against the physical effects to be avoided as a result of

excessive flow. In general gas flow rates of about 1 cu. ft./min. to about 20 cu. ft./min. are expected to be effective. Of course, reactor size and volume of the reaction mixture would also be factors to be considered in determining an appropriate gas flow rate.

In conducting the reaction of step b), the higher carboxylic acid is mixed with the product of step a) and the temperature of the mixture is increased until the by-product lower organic acid is evolved as a vapor. When acetic anhydride is a reactant in step a), evolution of acetic acid vapor is observed in step b) at a temperature of above about 150°C. The temperature is continually increased until the reaction is substantially complete and the upper temperature can be about 300°C. When the reaction of step b) is substantially complete, it is advantageous to reduce the pressure of the reaction in order to produce as complete removal as possible of by-product lower organic acid and the attendant completeness of reaction. Of course, it is possible to conduct the entirety of reaction step b) under a sub-atmospheric pressure along with an inert gas passing through the mixture. The degree of pressure reduction is not particularly critical and its choice depends in part on the physical properties (e.g., boiling point) of the particular by-product species being removed. Thus a convenient pressure reduction can be easily determined by those having ordinary skill in the art.

The high temperature used to drive the reaction of step b) to completeness also serves to eliminate excess

and unreacted higher carboxylic acid which is also vaporized. At the completion of the reaction of step b), the product remaining in the reactor is antimony tricarboxylate essentially free of deleterious impurities and can be used as a hydrocarbon cracking catalyst passivator without further purification. Recovery of the product therefore involves merely removing the antimony tricarboxylate of the higher carboxylic acid from the reactor after completion of step b).

Hydrocarbon feedstock containing higher molecular weight hydrocarbons is cracked by contacting it at an elevated temperature with a cracking catalyst whereby light distillates such as gasoline are produced. However, the cracking catalyst gradually deteriorates during this process. One reason for this deterioration is the deposition of contaminating metals such as nickel, vanadium, and iron on the catalyst, resulting in increased production of hydrogen and coke and decreased catalyst activity for cracking. Furthermore, the conversion of hydrocarbons into gasoline is reduced by these metals.

In accordance with another embodiment of this invention, we have discovered that the adverse effects of nickel, vanadium or iron on cracking catalyst can be precluded or reduced by using with the cracking catalyst the antimony higher tricarboxylates prepared as described above.

An improved cracking catalyst may be prepared by contacting the catalyst with the tricarboxylate of antimony before, during or after use of the catalyst in a hydrocarbon cracking process.

The term "cracking catalyst" as used herein refers either to fresh or used cracking catalyst materials that are useful for cracking hydrocarbons in the absence of added hydrogen. The cracking catalyst can be any conventional cracking catalyst.

Such cracking catalyst materials can be any of those cracking catalysts conventionally employed in the catalytic cracking of hydrocarbons boiling above 400°F. (204°C.) for the production of gasoline, motor fuel, blending components and light distillates. These conventional cracking catalysts generally contain silica or silica-alumina. Such materials are frequently associated with zeolitic materials. These zeolitic materials can be naturally occurring, or they can be produced by conventional ion exchange methods such as to provide metallic ions which improve the activity of the catalyst. Zeolite-modified silica-alumina catalysts are particularly applicable in this invention. Examples of cracking catalysts into or onto which the mixed-ligand tricarboxylate of antimony can be incorporated include hydrocarbon cracking catalysts obtained by admixing an inorganic oxide gel with an aluminosilicate and aluminosilicate compositions which are strongly acidic as a result of treatment with a fluid medium containing at

least one rare earth metal cation and a hydrogen ion, or ion capable of conversion to a hydrogen ion. The unused catalytic cracking material employed will generally be in particulate form having a particle size principally within the range of about 10 to about 200 microns.

The catalytic cracking materials can vary in pore volume and surface area. Generally, however, the unused cracking catalyst will have a pore volume in the range of about 0.1 to about 1 ml/g. The surface area of this unused catalytic cracking material generally will be in the range of about 50 to about 500 $m^2/g$.

The modified catalyst of this invention consists essentially of a conventional cracking catalyst having a modifying or passivating amount of the antimony higher tricarboxylate prepared as defined herein deposited on or in such catalyst. Such "modifying amount" is that amount sufficient to preclude or reduce the adverse effects of the nickel, iron or vanadium metals. Generally, the amount of antimony compound added corresponds to the level of impurity metals in the hydrocarbon feedstock and a typical "modifying amount" is from about 0.005% to 5% by weight of catalyst calculated as antimony metal.

The manner in which the conventional cracking catalyst is contacted with the antimony compound is not critical. Fresh catalyst can be impregnated with the antimony compound. Also, the catalyst can be contacted with the antimony compound during the cracking process

by metering the antimony compound into the hydrocarbon feedstock being fed to the cracking process.

The cracking process of this invention is basically an improvement of a known cracking process carried out in the absence of added hydrogen. This process is improved in accordance with this invention by the use of a novel metal passivator as defined above. More specifically, a hydrocarbon feedstock is cracked into lighter hydrocarbon materials by contacting this feedstock with an unused catalytic cracking material and antimony higher tricarboxylate prepared as defined herein and in the absence of added hydrogen and recovering the cracked materials.

A preferred embodiment of the cracking process of this invention utilizes a cyclic flow of catalyst from a cracking zone to a regeneration zone. In this process, a hydrocarbon feedstock containing contaminating metals such as nickel, vanadium or iron is contacted in a cracking zone under cracking conditions and in the absence of added hydrogen with an antimony containing cracking catalyst as defined above; a cracked product is obtained and recovered; the cracking catalyst is passed from the cracking zone into a regeneration zone; in the regeneration zone the cracking catalyst is regenerated by contacting the cracking catalyst with a free oxygen-containing gas, preferably air. The coke that has been built up during the cracking process is thereby at least partially burned off the catalyst. The regenerated

cracking catalyst is reintroduced into the cracking zone.

Also, it is to be understood that the used cracking catalyst coming from the cracking zone before it is introduced into the regenerator is stripped of essentially all entrained liquid or gaseous hydrocarbons. Similarly, the regenerated catalyst can be stripped of any entrained oxygen before it reenters the cracking zone. The stripping is generally done with steam.

The specific conditions in the cracking zone and in the regeneration zone are not critical and depend upon several parameters such as the feedstock used, the catalyst used and the results desired. Those skilled in the art are thoroughly familiar with those specific conditions and they can be easily determined for the given parameters. Preferably and most commonly, the cracking and regeneration conditions are within the following ranges:

| | Cracking Zone |
|---|---|
| Temperature: | 800°F to 1200°F (427-649°C) |
| Pressure: | Subatmospheric to 3,000 psig |
| Catalyst/Oil Ratio: | 3/1 to 30/1, by weight |
| | Regeneration Zone |
| Temperature: | 1,000°F to 1800°F (538°C to 982°C) |
| Pressure: | Subatmospheric to 3,000 psig |
| Air: (60°F. 1 atm) | 100-250ft$^3$/lb coke (6.2-15.6 m$^3$/kg coke) |

The following examples further illustrate specific embodiments of this invention but are not to be considered as limiting the invention to the specifics involved.

Example 1 -

A three neck reaction flask was equipped with a stirrer, a heating mantle and a reflux condenser attached to a Dean-Stark trap. The flask was charged with 175 grams of antimony oxide, 212 grams of acetic anhydride and 125 grams of xylene. With stirring, the reaction mixture was heated to 120°C and maintained at that temperature for 3 to 4 hours. Then, 663.9 grams of neodecanoic acid (technical grade) was added and the reaction mixture heated to 195°C. During the heating, xylene was removed by distillation and at approximately 155°C, the evolution of acetic acid was observed and was removed. Heating continued to 195°C and when 195°C was reached, the reaction mixture was cooled and the antimony trineodecanoate yield was determined to be 72.6%.

The technical grade neodecanoic acid used is a product of Exxon Chemicals and is a highly branced multi-isomer mixture combination with a typical hydrocarbon-type odor and melting point of less than -40°C. The structure of these isomers is generically represented by

```
  R'
  |
R-C-COOH
  |
  R"
```

The sum of the carbon atoms for R, R' and R" in each isomer is eight. The Exxon technical grade neodecanoic acid employed has a typical isomer distribution as follows:

i) $R=CH_3$, $R'=CH_3$, $R''=C_6H_{13}$..........31%

ii) $R<C_6H_{13}$, $R'=CH_3$, $R''>CH_3$...........67%

iii) $R<C_6H_{13}$, $R'>CH_3$, $R''>CH_3$.......... 2%

Therefore, as the term is used herein, neodecanoic acid and neodecanoate esters include each of the several isomers of the technical grade material, either alone or in mixture.

Example 2

The apparatus described in Example 1 was charged with 175 grams of antimony oxide, 212 grams of acetic anhydride and 125 grams of xylene. With stirring the reaction mixture was heated to 120°C and maintained at that temperature for 3 to 4 hours. Then, about 664 grams of neodecanoic acid (technical grade) was added and the reaction mixture heated to 200°C. During the heating from 120°C to 200°C, nitrogen was bubbled through the reaction mixture by use of a subsurface nitrogen sparge. While the reaction mixture was heated from 120°C to 200°C, xylene was removed by distillation and evolution of acetic acid was observed at about 155°C and was removed. When the temperature of 200°C was reached, the reaction mixture was cooled and the antimony trineodecanoate yield was determined to be 90%.

Example 3

The procedure of Example 2 was repeated with the exception that the reaction mixture was heated to 225°C after adding the neodecanoic acid reactant. After reaching 225°C, the reaction mixture was cooled and the antimony trineodecanoate yield was determined to be 94.2%.

Example 4

A Midwestern United States refinery typically uses thirty gallons per day of a commercial antimony F.C.C. catalyst aide with a hydrocarbon feed rate to the unit of 20,000 barrels per day. The catalyst aide was a colloidal dispersion of $Sb_2O_5$ in a hydrocarbon carrier. The refiner was charging 30-35 tons of fresh catalyst per day while maintaining a constant catalyst inventory of approximately 300 tons of catalyst and targeting for a hydrogen to methane molar ratio ($H_2$:$CH_4$) of 1.0. The concentration of nickel on the catalyst was determined to typically be 2000-3500 ppm. Typical analyses for antimony present on the catalyst and the determination of the $H_2$:$CH_4$ ratio are shown below:

| Antimony Charged (llbs. per day) | Antimony Found on catalyst (ppm) | Lay-down Efficiency (%) | $H_2$:$CH_4$ Ratio |
|---|---|---|---|
| 69 | 500 | 47 | $\geq$1.0 |

Example 5

The antimony tricarboxylate product of Example 2 was subjected to field evaluation in a typical Midwestern United States refinery as in Example 4. The

initial charge of 30 gallons per day of antimony tricarboxylate replaced the catalyst aide of Example 4. Analyses for antimony present on the catalyst and the determination for the $H_2$:$CH_4$ ratio were run after fourteen days. Additional data was collected after approximately two months time wherein the feed-rate of chemical had been lowered to 25 gallons per day. Those results are shown below:

| Time (days) | Sb Charged (llbs. per day) | Sb Found on Catalyst (ppm) | Lay-down Efficiency (%) | $H_2$:$CH_4$ Ratio |
|---|---|---|---|---|
| 14 | 59 | 790 | 86 | 0.9-1.0 |
| 16 | 49 | 700 | 92 | 1.0 |

As seen by this example, the feed-rate of the antimony catalyst aide can be expected to decrease 28.57% with a lay-down efficiency increase of approximately 2-fold while maintaining the $H_2$:$CH_4$ ratio in the desired range as compared with the aide of Example 2.

Example 6

The Midwest refinery of this example was charging 15,000 barrels per day of hydrocarbon feedstock to the F.C.C. unit while maintaining a constant catalyst inventory and fresh catalyst make-up. A commercial catalyst aide, $Sb_2P_5$ was in use which contained 11 to 12% antimony, a density of 10 lbs. per gallon and at a feed-rate of 5 gallons per day. The catalyst regeneration unit has a maximum operating temperature of 1400°F. The measure of performance of the catalyst aide was based upon regenerator temperature, antimony found on

the catalyst and the hydrogen to methane molar ratios. Typical results are presented below for both the commercial catalyst side and the antimony tricarboxylate from Example 2:

| Catalyst Aide | lbs. Sb Fed per day | Antimony Found on Catalyst (ppm) | Regeneration Temp. (°F) | $H_2:CH_4$ Ratio |
|---|---|---|---|---|
| Commercial | 11.5 | 400 | 1370 | $\geq$1.0 |
| Product Ex. 2 | 9.89 | 1100 | 1350 | 0.8-0. |
| Product Ex. 2 | 7.91 | 800 | 1350 | 1.0 |

CLAIMS

1. A method of producing higher tricarboxylates of antimony comprising the steps of

(a) forming an antimony tricaboxylate by reacting antimony oxide with an anhydride of a lower organic acid at an elevated temperature and optionally in the presence of a solvent,

(b) reacting the product from (a) with at least one higher carboxylic acid at a temperature sufficiently high to replace substantially all of the lower organic acid radicals with higher carboxylic acid radicals and remove the lower organic acid by-product and unreacted higher carboxylic acid from the reaction zone, the reaction of (b) optionally being effected while simultaneously passing a stream of inert gas through the reaction mixture, and

(c) recovering the antimony tricarboxylate of said higher carboxylic acid.

2. A method according to claim 1 wherein the anhydride of a lower organic acid has the formula $R_1$-O-$R_1$ wherein $R_1$ is alkanoyl of not more than 5 carbon atoms and the or each higher carboxylic acid has the formula $R_2COOH$ wherein $R_2$ is alkyl of at least six carbon atoms.

3. A method according to claim 2 wherein the higher carboxylic acid comprises neodecanoic acid or a mixture of

two molar parts of 2-ethylhexanoic acid and one molar part of neodecanoic acid.

4. A method according to any one of claims 1 to 3 wherein the reaction in step (b) is at about 150°C to 300°C.

5. A method according to any one of claims 1 to 4 wherein the anhydride of a lower organic acid is acetic anhydride.

6. A method according to any one of claims 1 to 5 wherein step (a) is in the presence of a hydrocarbon having a boiling point of at least 120°C, as solvent.

7. A method according to claim 6 wherein the solvent is selected from toluene, cumene, xylene and kerosene.

8. A process for the catalytic cracking of a hydrocarbon feedstock in a cracking zone under cracking conditions essentially in the absence of added hydrogen in the presence of a zeolite-containing cracking catalyst and an antimony compound catalyst aide, with said feedstock containing at least one catalyst contaminating metal selected from the group consisting of nickel, vanadium, iron, copper, and cobalt, and wherein optionally said cracking catalyst is removed from the cracking zone to a regeneration zone wherein the catalyst is contacted with free oxygen-containing gas to burn off coke deposited thereon, and the regenerated cracking catalyst is then recycled into the cracking zone, characterised in that said antimony compound comprises a higher tricarboxylate of antimony prepared by a process as claimed in any one of claims 1 to 8 wherein the lower organic acid has up to five carbon atoms and said at least one higher carboxylic acid has 6 to about 24 carbon atoms and the temperature of step (b) is sufficient to vaporise volatile material.

9. A process according to claim 8 wherein the amount of the higher tricarboxylate of antimony used is 0.005 to 5% by weight of catalyst calculated as antimony metal.

10. A process according to claim 8 or claim 9 wherein the higher tricarboxylate of antimony is antimony trineodecanoate or antimony di-2-ethylhexanoate neodecanoate.

11. A process according to any one of claims 8 to 10 wherein the zeolite-containing cracking catalyst contains an alumino-silicate zeolite.

12. A process according to any one of claims 8 to 11 wherein the zeolite-containing catalyst is combined with said higher tricarboxylate of antimony prior to contact with said hydrocarbon feedstock.

European Patent Office

# EUROPEAN SEARCH REPORT

0180675

Application number

EP 84 30 7780

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.4) |
| E | US-A-4 488 997 (MILLER et al.) * Claims 1,2; column 1, lines 23-28; column 2, line 63 - column 3, line 15, lines 54-61 * | 1-12 | C 07 C 51/41<br>C 07 C 53/128//<br>C 10 G 11/02 |
| E | US-A-4 488 998 (MILLER et al.) * Claims 1-14; column 1, lines 25-31; column 2, line 67 - column 3, line 19, lines 34-51; column 4, lines 27-34 * | 1-12 | |
| A | US-A-3 211 768 (CONSIDINE) * Claims 1-11, 13-15,17 * | 1-5 | |
| | | | TECHNICAL FIELDS SEARCHED (Int. Cl.4) |
| | | | C 07 C 51/00<br>C 07 C 53/00<br>C 07 F 9/00 |

The present search report has been drawn up for all claims

| Place of search | Date of completion of the search | Examiner |
|---|---|---|
| THE HAGUE | 02-07-1985 | KLAG M.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82